# EUROPEAN PATENT SPECIFICATION

(11) **EP 0 509 703 B1**
(45) Date of publication and mention of the grant of the patent: **04.06.1997**
(21) Application number: 92303070.4
(22) Date of filing: 07.04.1992
(51) Int. Cl.: A61F 13/02

(54) **Absorbent, moisture transmitive occlusive dressing**
Absorbierender,feuchtigkeitsdurchlässiger, okklusiver Verband
Pansement oclusif absorbant et permeable aux vapeurs

(30) Priority: 08.04.1991 US 682034
(43) Date of publication of application: 21.10.1992
(73) Proprietor: CHICOPEE, New Brunswick New Jersey 08903 (US)
(72) Inventor: Watson, Mary C., Dayton, New Jersey 08810 (US)
(74) Representative: Mercer, Christopher Paul

(56) References cited:
- EP-A- 0 249 694
- EP-A- 0 340 945
- WO-A-90/03155
- US-A- 4 909 244

## Description

### Field of the Invention

The invention relates to dressings for wound care and in particular to occlusive absorbent moisture transmissive dressings.

### Background of the Invention

The two absorbent structures most commonly used in wound care materials are either textile (woven or nonwoven) or hydrophilic polymers (gels or colloids). Efficacious absorbency for textile absorbent products is considered to be 100% to 800% per unit weight. Absorbent capacity for commercial wound care gel dressing is commonly 200% to 300% per unit weight.

Historically, two problems with textile dressings have continued to defy solution. Difficulty is frequently encountered removing these materials from the wound without disturbing the healing that has taken place. In addition, these products are capable of depositing fiber debris in the wound, possibly resulting in the formation of granulomas and poor wound healing.

A similar difficulty exists with the gel dressings in that they frequently have insufficient integrity after use to be removed from the wound in tack. Gels frequently break up on removal and hence have the propensity to contaminate the wound.

Neither textile nor gel dressings have proven to be effective in occluding wound sites. Occlusion requires the sealing of the wound area so as to prevent airborne infection from entering the wound area.

U.S. Patent 4,738,257 discloses an occlusive dressing for care of skin wounds which includes a skin contacting barrier layer and an overlying backing layer. The backing layer is stretchable and the barrier layer having an elastic phase integrated by a cross-linked polymer network with particular water-absorbing hydrocolloid phase dispersed therein. The hydrocolloids disclosed include carboxymethylcellulose (CMC), sodium CMC, karaya, gelatin and guar. A partially open cell foam may be used as the backing layer in order to provide some vapor transmission.

U.S. Patent 4,595,001 discloses a surgical dressing which consists essentially of a film which carries an adhesive layer for securing the dressing to the body. The adhesive layer is adapted to allow access of water to the film. The dressing has a MVP (moisture vapor permeability) of not more than 2000 g/m² when the adhesive is in contact with moisture vapor but not water.

U.S. Patent 4,747,401 describes a surgical dressing which consists essentially of a film and structure similar to that of the '001 patent above. However, the '401 patent has a MVP of not less than 2500 g/m² when the adhesive layer is in contact with water and a MVP of not more than 2000 g/m² when the adhesive is in contact with moisture vapor but not water.

U.S. Patent 4,759,354 describes a wound dressing having a vapor permeable layer with an adhesive layer for adhering to the skin and acting as a reservoir for exudate. A layer of collagen is provided for direct contact with the wound.

Finally, U.S. Patent Nos. 4,318,408 and 4,449,977 describe a flexible absorbent product and compositions for the preparation of such articles. The product has a water-insoluble substantially non-swelling matrix of an elastomeric polymer having a uniformly dispersed particulate water-insoluble water-swellable organic polymer absorbent.

U.S. Patent No. 4,901,714 assigned to E. R. Squibb & Sons, Inc. describes a bandage having a layer of plastic, either in film or coating bonded to a sheet of nonwoven material to form a base. The plastic film is coated with an adhesive. The superabsorbent pad, smaller than the base, is wrapped in nonwoven material and affixed to the base by the exposed adhesive.

### Summary of the Invention

According to the present invention there is provided a wound dressing as defined in the claims.

The invention provides for an occlusive translucent wound dressing which has a moisture vapor permeable water impervious backing sheet, an absorbent layer of polymer matrix, and a peripheral adhesive coating for attachment about the wound site. The absorbent layer has dispersed therein an absorbent water-swellable, water-soluble material and the absorbent layer has an absorptive capacity of from 400% to 800% by weight. There are a variety of commercial backing films available which may be selected to produce a desired moisture vapor transmission rate (MVTR).

It has been found that the optimum MVTR for wound healing is in the range recited above. Too low an MVTR would keep the wound site too moist and actually cause maceration to the area surrounding the wound site. An MVTR which is too high would keep the wound site too dry and, therefore, make it less hospitable to healing.

The occlusive wound dressing may advantageously be formed with an elastic polymer forming the polymer matrix and having therein a water soluble absorbent material such as guar gum or carboxylmethylcellulose. The elastic polymer may preferably be a styrene-isoprene-styrene block copolymer thus providing elasticity and conformability of the absorbent layer to best promote the absorption and transmission of fluid from the wound and the occlusion of the wound site.

Preferably, the absorbent material should be present in a quantity of from 50% to 150% by weight of the polymer matrix. The wound dressing according to the invention is non-adhering to the wound site while providing adequate MVTR, absorption, wound occlusion and wound inspection.

The dressing of the invention provides a new and improved wound care product which surpasses products previously known. The product of the present invention provides a transparent or at a minimum a translucent dressing which permits visual inspection of the wound. The dressing further provides a clean release, that is, the dressing avoids the propensity of previous textile and gel dressings to leave fibers and/or particles at the wound site upon removal. The present occlusive dressing provides a clean release and remains substantially intact, thus avoiding the propensity of hydrocolloids to sluff off and remain in the wound site. The absorbent layer absorbs the wound exudate and reduces the pooling of exudate. Pooling, i.e. the amassing of a quantity of exudate, can cause dressing failure by causing a passageway to be formed under the dressing adhered to the wound and/or under the adhesive holding the dressing to the skin. The passageway formed violates the occlusive dressing cover and provides an entry for infection.

### Brief Description of the Drawings

Description of the invention will now be given with reference to the accompanying drawings wherein:
FIGURE 1 is a bottom plan view of an occlusive wound dressing of the invention;
FIGURE 2 is a cross-section along line 2-2 of FIG. 1.

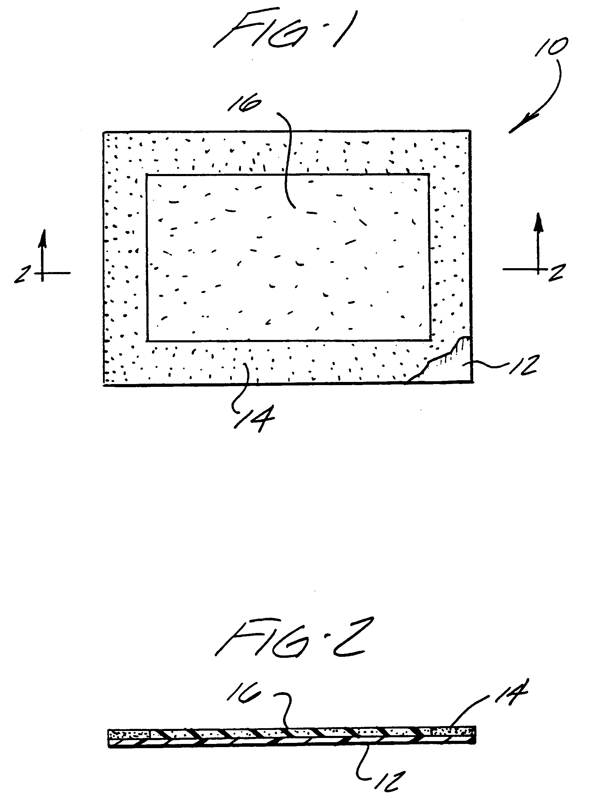

### Detailed Description of the Invention

The invention provides an occlusive dressing 10 having a backing layer 12 which could be a film of polyurethane, polyetherester or polyetheramide material. The backing layer is moisture vapor permeable but water impermeable. This layer could be chosen, for example, so as to provide a hydrated MVTR of about 2500 g/m²/24 hours to the completed dressing. A film of 1 to 3 mils thickness made of PEBAX (sold by Atochem Inc.) polyetheramide has been shown to be appropriate. Other commercially available backing films may be used to provide different MVTR's.

An absorbent layer 16 is provided in face-to-face engagement with the backing layer 12. This absorbent layer is preferably cast from a styrene-isoprene-styrene copolymer such as Kraton 1102 and 1107 (sold by Shell Chemical Co. ) in a toluene solvent. The absorbent layer preferably does not extend to the edges of the backing layer.

About the periphery of the dressing is an adhesive 14 such as an acrylic, a rubber-base, or polysiloxane. Preferably the adhesive is a pressure sensitive adhesive capable of attaching the dressing to the human body without such attachment force to cause injury upon removal. Within the absorbent layer there may be dispersed therein a sufficient quantity of a water-swellable water-soluble substance such as guar gum. This substance may act to provide the absorbent characteristics to the absorbent layer 16. Such a layer may be made by mixing the absorbent substance with a solvent solution of the baton and solvent casting. Alternatively, the absorbent may be added to a polymer melt and extruded.

Alternative embodiments of the absorbent layer may be prepared by dispersing the water-swellable, water-soluble material in a hydrophilic polymer such as a modified polyvinyl alcohol (PVOH), Vinex 33 (sold by Air Products Co.) for example. The invention further includes an alloy or a copolymer resin made from hydrophilic and hydrophobic polymers.

The water-swellable, water-soluble material in the absorbent layer may be a natural or modified naturally occurring polymer such as sodium alginate, alkyl and hydroxyalkylcelluloses, carboxylmethylcellulose or hydroxylalkylcellulose. Also found to be useful are gums such as guar gum, agar, pectin, locust bean, karaya and ghatti. Further substances such as synthetic absorbent polymers are also useful as, for example, polyacrylamide, poly(acrylic acid) and homologs, polyethyleneglycol, polyethyleneoxide, polyvinylalcohol and polyvinylpyrrolidone.

The resulting dressing provides an absorption layer next to the wound to act as a reservoir and vapor transmission through the backing layer to assist in wound healing. All of this is provided in a transparent dressing permitting wound inspection.

### EXAMPLES

Example 1 is the preparation of translucent absorbent layer containing carboxymethylcellulose (CMC). Suitable CMC's are those such as cellogen sold by DKS International.

CMC ABSORBENT (100% rubber solids) was uniformly mixed with the following formulation:

| | |
|---|---|
| Kraton® 1107 | 24.5% |
| Tinuvin 328 (or Irganox MD 1024) | 0.1% |
| Irganox 1035 | 0.1% |
| Methyl Paraben | 0.1% |
| Toluene | 75.0% |

100% rubber solids means that the CMC added was equal in weight to the weight of the rubber solids.

Films were prepared by solvent casting from toluene solutions of 25% Kraton® rubber resin. Rubber resin, antioxidants {Tinuvin 328; 2(2′-hydroxy-3,5′-di-tect-amylphenyl) benzo-triazole} and the preservative (methyl paraben) were dissolved in toluene and mixed on a roller mixer for forty-eight hours. CMC was added to the solvent solution and thoroughly mixed to obtain a uniform suspension. The formulation was coated on release paper with a gardner knife having a 10 mil gap.

The samples were dried in a hood and placed in an oven at 200°F for 30 seconds to remove any residual solvent. The GAT method, as described in U.S. Patent No. 4,901,714, the disclosure of which is incorporated herein by reference, of saline absorbency was chosen for sample evaluation because it more closely simulates the end use application than total immersion of the substrate. MVTR was measured using a Mocon tester at 37°C and 100% RH.

The samples had an average absorbency of 8.5 g/g and an average MVTR of 4657 gms/m²/24 hours.

Example 2 uses a water-swellable water-soluble absorbent, guar gum, as the absorbent. Guar gum the AA (100% rubber solids) was uniformly mixed with the following formulation:

| | |
|---|---|
| Kraton® 1107 | 24.5% |
| Tinuvin 328 | 0.1% |
| Irganox 1035 | 0.1% |
| Irganox 1024 | 0.1% |
| Methyl Paraben | 0.1% |
| Toluene | 75.0% |

The absorbent layer was prepared as above. The absorbent layer had an average absorbency of 4.1 gms/gm and a MVTR of 2500 gms/m²/24 hours for a hydrated sample and 1500 gms/m²/24 hours for a dry sample.

The absorbent layer of Example 2 was applied to a backing layer of PEBAX copolymer (medifilm 810). The combined backing and absorbent layer had an average MVTR of 1200 gm/m²/24 hours when dry and an average MVTR of 2430 gm/m²/24 hours when hydrated.

## Claims

1. A wound dressing (10) comprising a water impervious backing sheet (12) and an absorbent layer (16) of polymer matrix having dispersed therein an absorbent water-swellable material and characterised in that the absorbent layer (16) is non-tacky and translucent and the backing sheet (12) is transparent to enable visual inspection of the wound, and a margin area (14) of the backing sheet (12) having an applied adhesive for attachment of the dressing over the wound.

2. The wound dressing (10) according to claim 1, wherein said polymer matrix is formed in part of an elastic polymer.

3. The wound dressing (10) according to claim 2, wherein said elastic polymer is a styrene-isoprene-styrene block copolymer.

4. The wound dressing (10) according to claim 3, wherein said absorbent material is chosen from the group guar gum, agar, ghatti, karaya, locust bean, pectin, traganth.

5. The wound dressing (10) according to claim 1, wherein said absorbent material is a synthetic absorbent polymer chosen from the group polyacrylamide, poly(acrylic acid) and homologs thereof, polyethyleneglycol, polyethyleneoxide, polyvinyl alcohol, and polyvinylpyrrolidone.

6. The wound dressing (10) according to claim 1, wherein said absorbent material is chosen from the group sodium alginate, hydroxyalkylcellulose, and carboxymethylcellulose and sodium alginate.

7. The wound dressing (10) according to any of the Claims 4, 5 or 6 wherein said absorbent material is present in a quantity from 50% to 150% by weight of said polymer matrix.

8. The wound dressing (10) according to Claim 7 wherein said absorbent material and said polymer matrix are each about 50% by weight of said absorbent layer.

9. The wound dressing (10) according to Claim 1 wherein said absorbent layer (16) is non-adhering to the wound site when hydrated.

## Patentansprüche

1. Wundverband (10), umfassend eine wasserundurchlässige Unterlage (12) und eine absorbierende Schicht (16) einer polymeren Matrix, in der ein absorbierendes, in Wasser quellbares Material dispergiert ist, dadurch gekennzeichnet, daß die absorbierende Schicht (16) nicht-klebrig und durchscheinend ist und die Unterlage (12) durchsichtig ist, um eine Sichtprüfung der Wunde zu ermöglichen, und daß ein Randbereich (14) der Unterlage (12) vorgesehen ist, auf den ein Klebstoff zur Befestigung des Verbands auf der Wunde aufgebracht ist.

2. Wundverband (10) nach Anspruch 1, wobei die polymere Matrix teilweise aus einem elastischen Polymeren gebildet ist.

3. Wundverband (10) nach Anspruch 2, wobei es sich beim elastischen Polymeren um ein Styrol-Isopren-Styrol-Blockcopolymeres handelt.

4. Wundverband (10) nach Anspruch 3, wobei das absorbierende Material aus der Gruppe Guargummi, Agar, Ghatti, Karaya, Johannisbrotkernmehl, Pektin und Traganth ausgewählt ist.

5. Wundverband (10) nach Anspruch 1, wobei es sich beim absorbierenden Material um ein syrithetisches absorbierendes Polymeres handelt, das aus der Gruppe Polyacrylamid, Poly-(acrylsäure) und Homologe davon, Polyethylenglykol, Polyethylenoxid, Polyvinylalkohol und Polyvinylpyrrolidon ausgewählt ist.

6. Wundverband (10) nach Anspruch 1, wobei das absorbierende Material aus der Gruppe Natriumalginat, Hydroxyalkylcellulose und Carboxymethylcellulose sowie Natriumalginat ausgewählt ist.

7. Wundverband (10) nach einem der Ansprüche 4, 5 oder 6, wobei das absorbierende Material in einer Menge von 50 bis 150 Gew.-% der polymeren Matrix vorhanden ist.

8. Wundverband (10) nach Anspruch 7, wobei das absorbierende Material und die polymere Matrix jeweils etwa 50 Gew.-% der absorbierenden Schicht ausmachen.

9. Wundverband (10) nach Anspruch 1, wobei die absorbierende Schicht (16) im hydratisierten Zustand nicht an der Wundstelle haftet.

## Revendications

1. Un pansement chirurgical (10) comprenant une feuille support imperméable (12) et une couche absorbante (16) composée d'une matrice polymère présentant à l'intérieur de celle-ci une matière absorbante qui gonfle au contact de l'eau, et caractérisé en ce que la couche absorbante (16) est non adhésive et translucide, et en ce que la feuille support (12) est transparente de sorte qu'il est possible de réaliser un examen visuel de la plaie, et en ce qu'il existe une zone marginale (14) de la feuille support (12) sur laquelle est appliqué un adhésif permettant de fixer le pansement sur la plaie.

2. Le pansement chirurgical (10) selon la Revendication 1, dans lequel ladite matrice polymère se compose pour partie d'un polymère élastique.

3. Le pansement chirurgical (10) selon la Revendication 2, dans lequel ledit polymère élastique est un copolymère bloc de styrène-isoprène-styrène.

4. Le pansement chirurgical (10) selon la Revendication 3, dans lequel ladite matière absorbante est choisie parmi le groupe composé de gommes de guar, d'agar, de ghatti, de sterculia, de graine de caroube, de pectine et d'adragante.

5. Le pansement chirurgical (10) selon la Revendication 1, dans lequel ladite matière absorbante est un polymère absorbant synthétique choisi parmi le groupe composé de polyacrylamide, d'acide poly(acrylique) et de leurs homologues, polyéthylèneglycol, oxyde de polyéthylène, alcool de polyvinyle et pyrrolidone de polyvinyle.

6. Le pansement chirurgical (10) selon la Revendication 1, dans lequel ladite matière absorbante est choisie parmi le groupe composé d'alginate de sodium, d'hydroxyalkylcellulose et de carboxyméthylcellulose.

7. Le pansement chirurgical (10) selon l'une des Revendications 4, 5 ou 6, dans lequel ladite matière absorbante est présente dans une proportion comprise entre 50 % et 150 % en masse de ladite matrice polymère.

8. Le pansement chirurgical (10) selon la Revendication 7, dans lequel ladite matière absorbante et ladite matrice polymère constituent chacune environ 50 % en masse de ladite couche absorbante.

9. Le pansement chirurgical (10) selon la Revendication 1, dans lequel ladite couche absorbante (16), lorsqu'elle est hydratée, n'adhère pas à la zone de la plaie.
